# EUROPEAN PATENT SPECIFICATION

(11) **EP 1 385 691 B1**
(45) Date of publication and mention of the grant of the patent: **27.10.2010**
(21) Application number: 01992638.5
(22) Date of filing: 01.11.2001
(51) Int. Cl.: B29C 67/20, A61F 2/06

(54) **METHOD OF MANUFACTURE OF NON-EXPANDED POROUS POLYTETRAFLUOROETHYLENE (PTFE) PRODUCTS**
HERSTELLUNGSVERFAHREN FÜR PRODUKTE AUS NICHT EXPANDIERTEM, PORÖSEM POLYTETRAFLUORETHYLEN (PTFE)
PROCEDES DE FABRICATION DES PRODUITS EN POLYTETRAFLUORETHYLENE (PTFE) POREUX NON EXPANSE

(30) Priority: 02.11.2000 US 704494
(43) Date of publication of application: 04.02.2004
(73) Proprietor: Boston Scientific Limited, Barbados, West Indies (BB)
(72) Inventor: GIRTON, Timothy, Samuel, Maple Grove, MN 55311 (US); SOGARD, David, John, Edina, MN (US)
(74) Representative: Hauck Patent- und Rechtsanwälte
(86) International application number: PCT/US2001/045550
(87) International publication number: WO 2002/036332

(56) References cited:
- EP-A2- 0 815 806
- WO-A-00/30564
- WO-A-87/02996
- WO-A-98/38947
- US-A- 4 657 544

## Description

### FIELD OF THE INVENTION

The present invention relates to a method of covering an endoprosthesis device with a non-expanded porous polytetrafluoroethylene (PTFE) product The non-expanded porous PTFE material is formed from an extruded mixture of PTFE resin and an extractable polymer material, the polymer material being extracted to leave voids or pores in the extrudate. Implantable tubular grafts, stent coverings, medical patches and fabrics can be made in this manner. Thin stent coverings can be applied on the exterior surface of the stent, on the interior surface of the stent, or both.

### BACKGROUND OF THE INVENTION

Porous PTFE is conventionally formed from a mixture of PTFE particles and lubricant which is pre-processed to form a compacted billet, extruded into a particular shape, such as a tube, and stretched or expanded to provide a node and fibril structure. Such a node and fibril structure not only imparts certain physical properties to products made therefrom, but also provides. porosity to the products. Once stretched it is referred to as expanded PTFE (ePTFE). Sintering of the ePTFE is then generally carried out to "lock in" the porous structure. In applications involving medical implants, such as grafts, stent-grafts, patches and other such products, expanded PTFE has provided appropriate porosity to allow assimilation of the implant by the body and tissue ingrowth into the porous wall, both of which are necessary for long term patency.

The formation of ePTFE requires a great deal of expertise and costly equipment. Parameters such as the ratio of lubricant to PTFE particles in the feed material, the pressure and temperature of extrusion, the temperature, rate and degree of stretching are some of the parameters which must be carefully controlled in order to achieve the desired characteristics in the final product. Even still porosity will vary in accordance with the fibril length, which for a given expanded structure may have a wide range.
WO-A-87/02996 describes a composition of interpenetrating matrices comprising a first polymer network characterized by nodes interconnected by fibrils and a second polymer network comprising diorganosiloxy units.

Other techniques of creating pores in polymeric materials have existed. For example, one known technique has been to include salt particles in polymer compositions, which salt particles are removed or leached out using water once the polymer is cured. Such techniques have also been applied to PTFE products. For example, U.S. Patent No. 4,576,608 discloses implantable products made from a compacted, sintered blend ofPTFE particles, PTFE fibers, a binder and a soluble salt, such as sodium chloride, which is leached out using water, leaving voids in the compacted resin. Similarly, U.S. Patent No. 4,849,285 discloses a matrix of unfibrillated PTFE resin and curable silicone in combination with particulate inorganic materials which remain in the PTFE structure to provide a self-supporting feature, thereby obviating the need for expansion. The addition of sodium chloride is disclosed as being useful for creating pores by dissolving the salt particles in water subsequent to sintering. No removal of the other components is suggested by this reference.

U.S. Patent No. 5,141,522 discloses a composite material for use with mammalian tissue which consists essentially of an unsintered microfibrillar, non-absorbable biocompatible component prepared from PTFE, a particulate bioabsorbable filler such as a lactide, carbonate, oxylate or lactone and a non-absorbable, biocompatible thermoplastic component. The bioabsorbable filler is incorporated into the expanded, porous structure of PTFE and is intended to be absorbed by the body over time. The non-absorbable thermoplastic component is intended to provide structural integrity to the material.

U.S. Patent No. 5,716,660 discloses an expanded PTFE tubular prosthesis having within its internodal spaces an insoluble, biocompatible material. The insoluble biocompatible material is introduced into the pores of the ePTFE using a dispersion or solution at acidic pH of biodegradable materials, which upon deposition into the porous structure of the ePTFE are rendered insoluble by increasing the pH. The biodegradable material within the pores is intended to encourage ingrowth and be absorbed by the body over time.

U.S. Patent No. 5,840,775 discloses a process for making porous PTFE by contacting PTFE with a fluid which penetrates and swells, but does not dissolve the PTFE. The fluid is introduced using temperature to permit extensive penetration of the PTFE. The liquid is then removed to leave a swelled, open structure of PTFE. As previously mentioned, expanded PTFE has been used extensively for grafts and stent-graft devices. Expanded PTFE covers and/or liners for stents have found to be particularly useful in endoprosthetic devices because while the porosity of the PTFE allows for assimilation of the device by the body, it also prohibits unwanted hyperplasia.

Endoprosthesis devices including stents, stent-grafts, grafts, vena cava filters, balloon catheters, and so forth, are placed or implanted within various body vessels for the treatment of various diseases. One particular type of an endoprosthesis device is the stent. A stent is implanted within a vessel for the treatment of stenoses, strictures, or aneurysms in the blood vessels. The devices are implanted within the vascular system to reinforce diseased, partially occluded, weakened or abnormally dilated sections of the blood vessel. Stents are often employed after angioplasty to prevent restenosis of a diseased blood vessel. While stents are most notably used in blood vessels, they have also been implanted in other bodily vessels including urinary tracts and bile ducts to reinforce and prevent neoplastic growth.

Stents are typically longitudinal tubular devices formed of biocompatible materials and come in a variety of construction types, and are often expandable in nature. Many if not all of the materials used for stents involve metal or carbon fiber materials which are highly electro-positive and are bio-active. Since stents tend to be used under conditions were they are counteracting disease processes, supporting healing processes, or guarding against stenosis of a passage, bio-activity, which may encourage undesirable or poorly regulated growth processes, or lead to clot formation, should be avoided.

Coating of the stent can keep the stent from directly contacting surrounding tissue or fluids, and thus can theoretically protect against unwanted electrochemically induced tissue reactions.

In the field of expandable stents, a further problem arises due to the fact that many stent constructions involve structures that have numerous apertures or spaces between various strands or structural elements of the stent such as those structures that are filamentous, wire-like, or of a tubular nature in which various openings have been cut or etched into the stent. With these constructions, tissue may grow through the openings of the stent. Furthermore, the stent itself may provoke a foreign body reaction and be both a stimulus for and a framework supporting, proliferative tissue growth, resulting, for example, in scar tissue or restenosis of the very region it is placed to control.

One approach to this drawback is to provide a coating, liner, cover or both, for the stent which prevents the healing or diseased layer of tissue from directly contacting the stent, or from passing through the stent in any way. Such liners may be formed, for example, of porous polytetrafluoroethylene (PTFE) which allows the passage of fluids and vital materials while serving as a barrier to tissue growth. However, when applying such a construction, a further difficulty which may arise is that the layer or sleeve of polymer must be attached to the stent for example, by staples or sutures at one end, or is prone to developing loose pockets or folds which might accumulate organic matter or lead to sepsis or unusual growth. Also, the necessarily thin liner material may detach or degrade. The risk of loose or unattached liner material is particularly great for constructions which utilize poorly adherent polymers, such as PTFE, or structures which seek to combine an expandable stent of stiff material, which changes both its dimension and its shape, with a dissimilar liner or shell.

One method for overcoming these problems is found in U.S. Patent No. 6,010,529 in which tube of polymeric material, e.g. expanded polytetrafluoroethylene (ePTFE), is passed through the interior of a stent body and is turned back upon itself over the stent to form a cuff. The assembly is then heated and the outer layer contacts and coalesces with the inner layer, closely surrounding the stent body within a folded envelope having a continuous and seamless end. Porosity is imparted to the PTFE by previous stretching or expansion the material.

Another type of covered stent which permits radial expansion is shown in WO 96/00103. As shown and described therein, a metallic expandable stent includes an outer covering of ePTFE. The ePTFE cover exhibits suitable expansion capabilities so as to enable the cover to expand upon expansion of the underlying stent. A polytetrafluoroethylene/lubricant blend may be extruded into a tube and the tube heated to remove the lubricant. Then, in order to impart the expandable characteristics to the ePTFE cover during formation of the ePTFE cover material, the ePTFE must undergo successive processing steps of expanding the material, sintering the material, radially dilating the material and resintering the dilated material, a procedure that is quite process intensive. The device described therefore requires precise manufacturing techniques and is extremely processing sensitive. Careful processing of the material forming the cover is required in order for the cover to exhibit sufficient expansion capabilities.
U. S. Patent No. 5,824,046 describes a composite intraluminal device, in particular an elongate radially expandable tubular stent having an interior luminal surface and an opposed exterior surface extending along a longitudinal stent axis. A stent cover is formed of unsintered ePTFE which is expandable.
EP 0 815 806 A2 discloses a covered stent for treating a stenotic region in a blood vessel. The stent covering has different porosities in different regions. In those regions, typically the ends, where tissue ingrowth and re-endothelialization are desired, the stent covering is more porous, and in those regions were it is desirable to inhibit such ingrowth, the stent covering is substantially non-porous.
WO 98/38947 discloses a method for forming a covered endoprothesis employing a conformed polymeric coating about an expandable stent. The expandable stent has an open tubular construction. A first polymeric liner is positioned about an inner surface of the tubular stent and an second polymeric liner is positioned about an outer surface of the tubular stent. The first and second polymeric liners are conformed to the tubular stent and laminated together through the open construction of the stent at a location coextensive with the inner surface of the tubular stent.
The problem of the invention is to provide a porous PTFE material which can be used in a variety of products and applications, and particularly in medical device applications, without requiring expansion to produce porosity.
The problem is solved according to claim 1.

### SUMMARY OF THE INVENTION

In one aspect of the present invention there is provided a method for producing an endoprosthetic device which includes a tubular extrudate having a PTFE matrix and distributed therein discrete domains of an extractable polymeric material, wherein upon exposure to sufficient dissolving medium or degradation temperature said discrete domains are extracted from said matrix to create pores in said tubular extrudate.

In another aspect of the present invention there is provided a method for producing a vascular graft comprising the aforementioned porous tubular extrudate.

In another aspect of the invention there is provided a method of forming a porous PTFE product which includes the steps of : providing a mixture of PTFE resin and an extractable polymer material; xtruding said mixture to form an extrudate which includes a PTFE matrix with discrete domains of said extractable polymer material; ubjecting said extrudate to a solvent for said extractable polymer material, a temperature sufficient to grade said extractable polymer material or a combination thereof, whereby at least a portion of said extractable polymer material is extracted, thereby forming pores in said extrudate.

In a stent-graft composite product, made by the method of the invention, the stent is radially distensible and has a cover, a liner or both at least partially covering the stent structure and being made from the porous PTFE material of the present invention. The stent can be affixed to the porous PTFE cover by any suitable means, including using adhesives, laminating inner and outer coverings through the stent openings, sutures, pockets or cuffs, or other such means.

Although the formation of pores in the products of the present invention are formed without expansion techniques, such porous PTFE materials may be subsequently subjected to conventional expansion and sintering processes.

The method of the invention is used to make an elongate radially expandable tubular stent having an interior surface and in exterior surface extending along a longitudinal stent axis. The expandable tubular stent has a stent cover on said interior surface, exterior surface or both, the cover being formed of a porous polytetrafluoroethylene. The porous polytetrafluoroethylene cover is a non-stretched (non-expanded) porous structure, the non-stretched structure lacking node and fibril structure.

In particular, the present invention relates to a method for producing a radially expandable stent for use in treating stenoses wherein the stent is at least partially covered with an expandable polymer covering which includes the porous PTFE of the present invention and which physically isolates the stent from surrounding blood and tissue.

According to the present invention a porous PTFE is prepared by extracting siloxane from an interpenetrating network (IPN) of PTFE and siloxane, leaving behind a porous PTFE structure without having to expand and stretch the PTFE.

### BRIEF DESCRIPTION OF THE DRAWINGS

Fig. 1 is a perspective view of one type of intraluminal stent device that may be made by the method of the present invention.

Fig. 2 is a perspective view of a different intraluminal stent device which may be made by the method of the present invention.

Fig. 3 is a perspective view of a stent-graft device which employs the intraluminal step device of fig. 1 in combination with a porous polytetrafluoroethylene cover made by the method of the present invention on both the inner and outer surface of the device.

Fig. 4 is a cross-sectional view of the same stent-graft device shown in Fig. 3.

Fig. 5 is the same stent-graft device as in Fig. 3 illustrating only the outer surface cover.

Fig. 6 is the same stent-graft device as in Fig. 3 with the exception that only a liner or inner surface cover is shown.

Fig. 7 is a cross-section of non-expanded PTFE extrudate having a PTFE resin matrix and extractable polymeric domains.

Fig. 7a is a cross-section of the non-expanded porous PTFE material of the present invention subsequent to removal of the polymeric domains..

Fig. 8 is a schematic representation of ePTFE node and fibril structure of the prior art.

### DETAILED DESCRIPTIONS OF THE PREFERRED EMBODIMENTS

In the method of the present invention the non-expanded porous PTFE materials are formed from a combination of a PTFE resin and an extractable polymeric component, which are admixed together and extruded to form an extrudate which includes a PTFE matrix having discrete domains of the extractable polymeric component distributed therein. The extractable polymeric component is desirably a particulate material of a particle size which facilitates admixing with PTFE resin powder. Desirably the extractable polymeric component is finely divided to a particle size of about 5 microns to about 100 microns. Distribution of the extractable polymeric component particles is largely determined by the degree of mixing prior to the extrusion process. Although not necessary, it is desirable that the PTFE resin particles and the extractable polymeric component are admixed prior to incorporation of the lubricant component generally associated with PTFE extrusion in order to facilitate uniformity of the mixture. Solvents and/or heat may be used to facilitate the mixing process.

Although the extractable polymeric component is desirably added to the PTFE resin in solid form, e.g. particulates, it is also possible to use liquid or gel forms of the polymer. For example, a gel, liquid or flowable form of an extractable polymeric component may be admixed with the PTFE resin. Such blending still results in separation of the extractable polymeric component from the PTFE matrix such that removal of the extractable polymeric component leaves a pore or void. An extractable polymer may also be incorporated into the PTFE resin in a solubilized or dispersion form, and admixed to form a composition which can be extruded into a product.

Once the aforementioned admixture is obtained, it may be subjected to conventional compaction into a billet for extrusion into tubular shapes, or subjected to other extrusion pre-conditioning depending on the final product application.

Extruded tubes made from the present compositions exhibit polymeric domains represented by the extractable polymers. The extrudate is then subjected to chemical fluids, (e.g. liquids or gases) thermal or electromagnetic radiation to remove the extractable polymer, leaving behind voids or pores, which generally have the shape of the extractable polymer which has been removed.

The extractable polymers are sufficiently incompatible with PTFE such that they naturally form domains upon extrusion. This is desirable since the formation of pores distributed in the PTFE matrix is intended. The choice of polymers may be selected from a wide range of materials, including those which are water-soluble and those which are water-insoluble. Combinations of polymers, with varying degrees of solubility may be employed.

The choice of solvent is dictated by the extractable polymer selected. Most solvents do not affect PTFE in any appreciable manner and its inertness is one of the important properties for use in medical devices. However, solvents which are known to deleteriously affect PTFE are not desirable. In some instances, the solubility of the extractable polymeric component in a given solvent may vary with temperature. Thus, the dissolving or extracting medium may be delivered with sufficient heat if necessary to maximize the ability to remove the polymeric domain. In some instances, such as when water or other medium which does not wet well the surface of PTFE, is used as the dissolvable medium, it may be necessary to include force, e.g. pressure or a combination of heat and force to penetrate to the polymeric domains.

Table I below provides a non-exclusive list of useful extractable polymeric components and selected solvents for their removal.

| **TABLE I** | |
|---|---|
| **Selected Extractable Polymers** | **Selected Useful Solvents** |
| polyvinyl alcohol (PVA) | water, methanol, ethyl alcohol |
| cellulose triacetate | acetone |
| poly(oxyethylene glycols) | water, toluene |
| ethyl cellulose | ethanol, isopropanol, methylacetate |
| methylcellulose | cold water |
| cellulose propionate | acetone, dioxane |
| carboxymethylcellulose | water |
| dextran (glucose polymer) | water |
| agar (poly(D-galactopyranose) | hot water |
| poly(vinylformal) | chlorinated solvents, aliphatic hydrocarbons |
| poly(sodium acrylic acid) | water |
| poly(sodium methacrylic acid) | water |
| sugar (polysaccharides) | water |
| polyvinylacetate | methanol, ketones, esters, chlorinated hydrocarbons, aromatic hydrocarbons |
| polystyrene | toluene |
| gelatin | water |
| wheat (prolamines) (simple proteins) | 75% alcohol |
| poly(vinylpyrrolidone) | water, organic solvents |
| poly(ethyleneterephthalate) | trifluoroacetic acid, o-chlorophenol, hexafluoroisoprophanol, various phenolics, phenol (cresol) |
| polyacrylonitrile (PAN) | dimethylformamide (DMF), dimethylsulfoxide (DMS), dimethylacetamide (DMAC), ethylene carbonate, propylene carbonate, adiponitrate, γ-butyrolactone |
| poly(methacrylate) (PMA) and poly(methylmethacrylate) (PMMA) | acetonitrate, isovinyl acetate, n-butylcholoride, heptanone-4, heptanone-3, n-propanol |
| poly(oxymethylene) | phenol (109°C) |
| | aniline (130°C) |
| | ethylene carbonate (145°C) |
| poly(acrylic acid) | alcohols, formamide, dimethylformamide |
| poly(acrylamide) | morpholine, water |
| nylon 6 | m-cresol, acetic acid, trichloroacetic acid |
| nylon 6,6 | formic acid, trifluoroethanol, chloral hydrate, dimethylsulfoxide (DMSO), formamide, acetic acid, chloroacetic acid |
| nylon 6,10 | chlorobenzene |
| nylon 11 | dimethylformamide, dimethylsulfoxide |
| nylon 18 | dimethylformamide, dimethylsulfoxide, pyridine |

In addition to water, organic solvents and other dissolving medium being employed to extract the polymer, the application of heat can be applied to cause the polymer to flow out of the PTFE matrix or to degrade the polymer such that it can be removed, for example, by subsequent dissolving medium. Additionally, electromagnetic radiation may be employed as a means of removing the polymer. Electron beam radiation is possible, but other forms may also be employed depending on the polymer.

In cases where the polymer is a crosslinked polymer, e.g. a thermoset polymer solvent dissolution may be difficult and degradation of the polymer may be a first step in its removal. In the case of thermoplastic polymers, extraction via solvents or heat is generally preferred. The molecular weight of the chosen polymer may vary extensively and its lower and upper ranges are only limited by practical concerns such as availability, difficulties in forming a blend with PTFE resin or difficulty in extraction.

It is generally known that PTFE resin has a density of 2.2 g/cc. As a result of undergoing the process of the present invention, porous, non-expanded PTFE products are formed having a bulk density desirably in the range of about 0.2 to about 0.5. Thus, a substantial amount of air space or porosity has been introduced into the PTFE material. The lower limit of porosity is governed by the need to establish sufficient ingrowth or acceptability in the body. The upper limit is governed by the need to maintain structural integrity of the porous PTFE product. Thus, a wide range of porosity is possible within these guidelines.

Porous, non-expanded PTFE products, and in particular non-expanded porous PTFE grafts are made in accordance with the present invention. These grafts may be used alone as surgical implants or combined with a supporting structure, such as a radially distensible stent, to form a stent-graft. Such stent-grafts are generally used as intraluminal devices, particularly in vascular applications.

The method of the present invention provides a covered stent which may be implanted intraluminally within a body vessel and disposed adjacent an occluded, weakened or otherwise damaged portion of the vessel so as to hold the vessel open. The covered stent is typically delivered intraluminally via a balloon catheter. The device is delivered in a compressed condition and once properly positioned may be deployed by radial expansion. The most common form of deploying the intraluminal device is by balloon expansion, however, the present invention may also be deployed by use of a self-expanding stent.

The stent may be made from a variety of materials including stainless steel, titanium, platinum, gold and other bio-compatible metals. Thermoplastic materials which are inert in the body may also be employed. Shaped memory alloys having superelastic properties generally made from specific ratios of nickel and titanium, commonly known as nitinol, are among the preferred stent materials.

Various stent types and stent constructions may be employed in the invention. Among the various stents useful include, without limitation, self-expanding stents and balloon expandable extents. The stents may be capable of radially contracting, as well and in this sense can best be described as radially distensible or deformable. Self-expanding stents include those that have a spring-like action which causes the stent to radially expand, or stents which expand due to the memory properties of the stent material for a particular configuration at a certain temperature. Nitinol is one material which has the ability to perform well while both in spring-like mode, as well as in a memory mode based on temperature. Other materials are of course contemplated, such as stainless steel, platinum, gold, titanium and other biocompatible metals, as well as polymeric stents.

The configuration of the stent may also be chosen from a host of geometries. For example, wire stents can be fastened into a continuous helical pattern, with or without a wave-like or zig-zag in the wire, to form a radially deformable stent. Individual rings or circular members can be linked together such as by struts, sutures, welding or interlacing or locking of the rings to form a tubular stent. Tubular stents useful in the present invention also include those formed by etching or cutting a pattern from a tube. Such stents are often referred to as slotted stents. Furthermore, stents may be formed by etching a pattern into a material or mold and depositing stent material in the pattern, such as by chemical vapor deposition or the like.

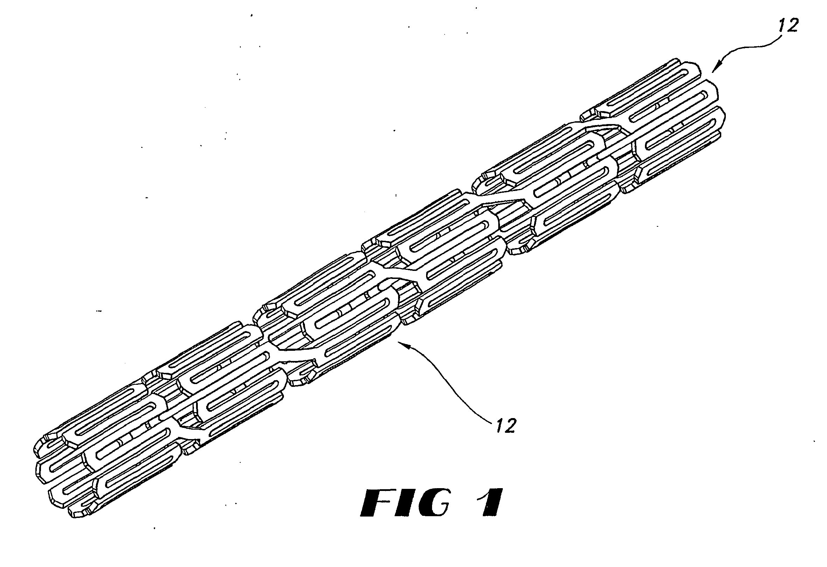

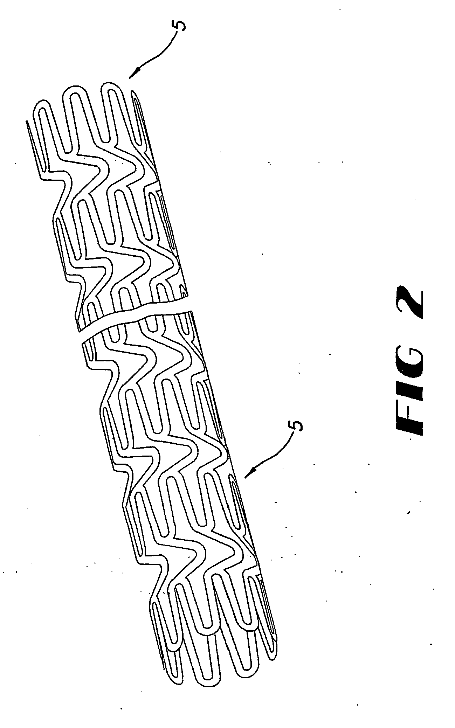

Fig. 1 illustrates an intraluminal device in the form of a stent 12. Fig. 2 illustrates an intraluminal device in the form of a stent 5 having a different construction than that shown in fig. 1.

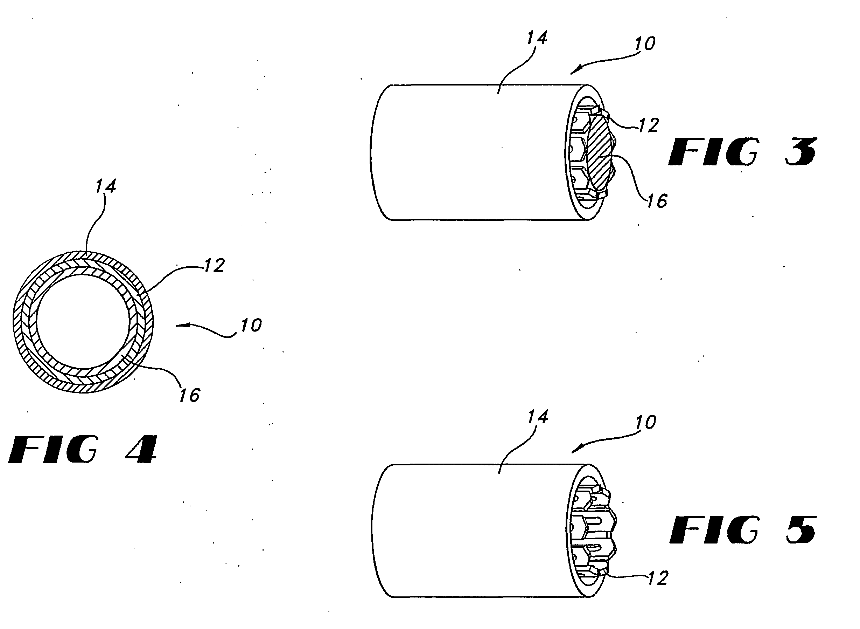

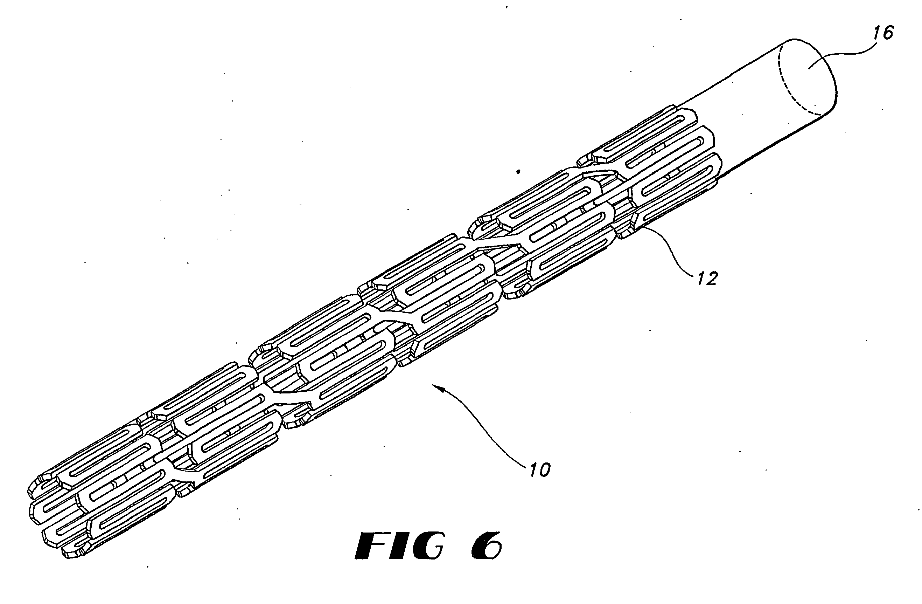

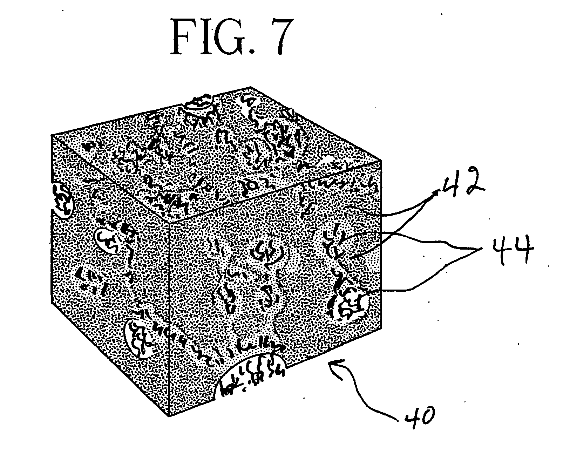

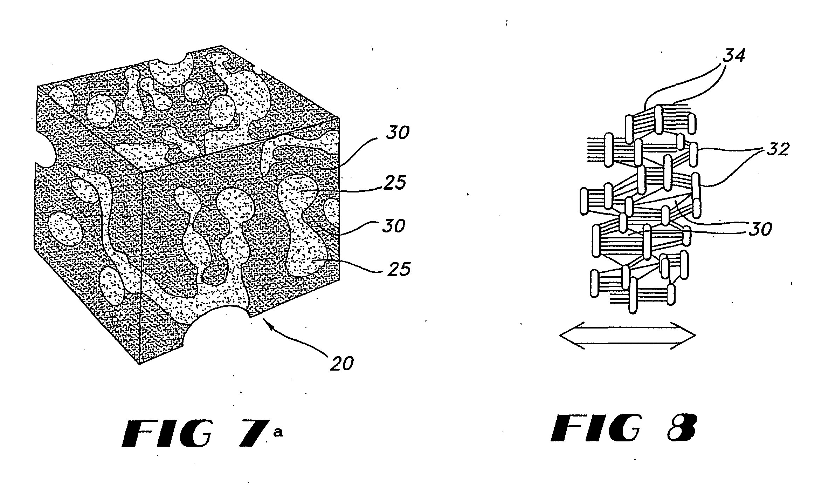

Fig. 3 illustrates generally at 10 an intraluminal device in the form of a stent 12 as shown in fig. 1 having a cover 14 on the outer surface 12 and liner 16 on the inner surface, both of which may be of the porous structure shown below in fig. 7. The stent may optionally have only a cover 14 as shown in fig. 5, or only a liner 16 as shown in fig. 6, or both as shown in fig. 3. In a preferred embodiment, the stent has both a cover 14 and a liner 16. The liner, cover, or both, will be referred to hereinafter collectively as a cover or covering. The cover provides an effective barrier about the stent 12 preventing excessive cell or tissue ingrowth or thrombus formation through the expanded wall of the stent 12.

Fig. 4 is a cross-sectional view of the same device as shown in fig. 3 with a cover 14 and a liner 16 around stent 12.

Fig. 1 is a more detailed illustration of stent 10 and shows generally an elongate tube. The body of stent 12 defines an opposed interior surface 11 and an exterior surface 13 and is formed of a generally open configuration having a plurality of openings or passages provided for longitudinal flexibility of the stent as well as permitting the stent to be radially expanded once deployed in the body lumen. Both the interior surface 11 and the exterior surface 13 may have the porous PTFE covering of the present invention. On the interior surface the covering is referred to as the liner 12 as shown in Fig. 1 and on the exterior surface it is referred to as a cover 14 as shown in Fig. 1.

While the figures illustrate a particular construction of stent 10, one of skill in the art would recognize that the porous PTFE covering material as described by the present invention would find utility in any stent configuration, and in particular the open stent configurations.
Stent 12 may be employed in combination with a cover 14 or liner 16 but is preferably employed with both. The cover 14 may be applied over the tubular stent 12 so as to fully circumferentially surround the stent 12, while the liner 16 is applied inside and through the stent 12 so that the stent 12 fully circumferentially surrounds the liner 16.
In one particular desirable embodiment of the present invention, the porous polytetrafluoroethylene (PTFE) material useful herein is first obtained in the form of an interpenetrating network of PTFE and a siloxane. In particular, polydimethylsiloxanes have been found to be useful. The silicone is then extracted from the IPN using either thermal or chemical means. The removal of the silicone leaves behind a porous PTFE structure. A particular material for use herein is SilonM), an interpenetrating polymer network (IPN) of polytetrafluoroethylene (PTFE) and polydimethylsiloxane (silicone) supplied by Bio Med Sciences, Inc. located in Bethlehem, PA. Such IPN polymer networks are described in U. S.Patent No. 6,022,902. In this patent, Silon^{®} is described as a breathable, hydrophobic polysiloxane membrane reinforced with poly (tetrafluoroethylene).
Fig. 7 shows a three-dimensional representation of an extrudate 40 which includes a PTFE resin matrix 42 having distributed therein discrete extractable polymeric domains 44. Such an extrudate may be extruded into a variety of shapes. Desirably, it is rolled or extruded into either a flat sheet, which is then formed into a tubular structrue for use as a graft or in a stent-graft device, or alternatively it is directly extruded into tubular form 14 as shown, in Fig. 5.
The removal of the extractable polymeric component from the IPN leaves behind a porous PTFE structure without having to go through the added steps of stretching or expanding the PTFE in order to obtain the porous structure. Quite obviously, this simplifies the manufacturing process by decreasing the number of steps required, and also increases efficiency. As previously described, porous PTFE often requires the expanding and stretching steps in order to achieve the desired porous structure. Fig. 7a illustrates generally at 20 a porous PTFE structure after removal of the extractable polymeric component. The removal of the polymeric domain leaves behind the porous structure wherein voids or pores 25, are found distributed within matrix PTFE 30.

The porous PTFE structure produced by the present inventive process is quite different from the porous structure produced by PTFE which has been stretched, or expanded. Typically, PTFE which has been stretched (ePTFE) has a node and fibril structure as seen in Figure 8. After stretching, the ePTFE possesses nodes 32 connected to fibrils 34. The spaced in between the nodes and fibrils represent pores 36.

When the extractable polymeric component is a siloxane, removing the siloxane from the IPN PTFE matrix of siloxane/PTFE through the use of heat involves heating the IPN structure to temperatures of between about 300°C and about 390°C. Alternatively, chemical removal of the siloxane may be accomplished using a compound selected from the group consisting of toluene, heptane, chloroform.

Sintering is typically accomplished at or above the crystalline melting point of PTFE. It refers to the bonding of particles in a mass by molecular (or atomic) attraction in the solid state through the application of heat below the melting point of the polymer. Sintering causes the strengthening of the powder mass and normally results in densification and often recrystallization.

A PTFE tube may be extruded as a tube from an extrusion device, or extruded as a film and subsequently wrapped into a tube. Extrusion techniques of PTFE are well known in the art.

As discussed above, the stent may be covered on the interior surface 11 of the stent 10, the exterior surface 13 of the stent 10, or both. Preferably, the stent 10 is covered on both the interior 11 and the exterior 13 surfaces of the stent 10. Having the entire surface of the stent 10 covered with the porous PTFE of the present invention provides an effective barrier about the stent 10 preventing excessive cell or tissue growth, or thrombus formation through the expanded wall of a tubular stent 10.

In order for the covering of porous PTFE to function effectively in combination with an expandable stent, the material must exhibit sufficient expansion characteristics so as to enable the stent cover to open or expand along with the radial expansion of the stent 10. If the covering is applied to the stent in its fully deployed state, and then folded during insertion, it may only require unfolding as the stent radially expands. The porous, non-expanded PTFE covering may additionally be subjected to expansion prior to attachment to the stent, or alternatively be affixed to the stent in the radially compressed state and expanded during the expansion of the stent. If the covering material does not effectively open or expand with the stent, several problems can arise. The covering material may tear, and may even detach from the surface of the stent if improper or dissimilar expansion of the covering material occurs with the expansion of the stent.

In order to improve the adhesion, and further prevent detachment of the PTFE covering from the stent, the PTFE may be fused or welded around or to the metal stent. This may be accomplished either through a heating process and/or bonding process. If heating is utilized, typically the PTFE will be heated above its sintering temperature.

If an adhesive is utilized, preferably a biocompatible adhesive is used. Such adhesives are known to one of skill in the art and include, for example, polyurethanes, epoxies, cyanoacrylates, polyamides, polyimides, silicones, and so forth. Dispersions of PTFE or FEP (fluoroethylpropylene) may also be utilized. This list is not exclusive and is intended for illustrative purposes only, and is in no way intended as a limitation on the scope of the present invention. There is a vast number of adhesives that can be used for such applications, limited by their biocompatibility, and by their ability to bond to polymeric materials (e.g. PTFE) and metals, particularly in aqueous environments.

The covering material may also be assembled to the intraluminal device in more than one piece. Such a combination would require overlapping of sorts of the PTFE material, and subsequent fusion or adhesive bonding of the porous PTFE material to itself.

It is preferable, however, to utilize the porous PTFE covering in a continuous form such as a membrane or thin film. The porous PTFE (after removal of the extractable polymeric component), in the form of a membrane or a thin film, thus, preferably completely wraps the metal stent, thereby providing a barrier that physically isolates the stent from surrounding blood and tissue. This barrier further helps prevent healing or diseased layers of tissue from directly contacting the stent, or from passing through the stent in any way. The porous PTFE allows the passage of fluids and vital materials, however, while still serving as a barrier to tissue growth.

## Claims

1. Method of covering an endoprosthesis device with porous PTFE product without a node and fibril structure (5; 12; 20), which method comprises the steps of:
- providing an elongate radially expandable tubular stent having an interior surface and an exterior surface extending along the longitudinal stent axis,
- providing a mixture of PTFE and an extractable polymer material;
- extruding said mixture to form an extrudate (40) comprising a PTFE matrix (42) with discrete domains of said extractable polymer material (44);
- subjecting said extrudate to a solvent for said polymer material, a temperature sufficient to degrade said polymer material or a combination thereof, said extrudate in the form of an interpenetrating network of polytetrafluoroethylene and the extractable polymer material, the extractable material being siloxane, whereby at least a portion of said polymer material is extracted, thereby forming porous polytetrafluoroethylene having pores (25) in said non-stretched extrudate (30) without a node and fibril structure,
- forming a stent cover from said porous PTFE, and
- applying said stent cover to the interior surface, exterior surface or both of said stent wherein said stent cover extends along the longitudinal stent axis.

2. Method according to claim 1, wherein the mixture is extruded as a tube.

3. Method according to claim 1, wherein the mixture is extruded as a film which is subsequently wrapped into a tube.

4. Method according to claim 1 wherein said siloxane is chemically extracted by a compound selected from the group consisting of toluene, heptane and chloroform.

5. Method according to claim 1 wherein said siloxane is removed by heating said network to a temperature of at least about 300° C.

6. Method according to claim 1 wherein said siloxane is polydimethylsiloxane.

7. Method according to claim 1 wherein said stent cover is applied to said interior surface and to said exterior surface of said stent.

8. Method according to claim 1 wherein said stent cover is fixed to said stent using an adhesive.

9. Method according to claim 8 wherein said adhesive is selected from the group consisting of polyurethanes, epoxies, cyanoacrylates, polyamdies, polyimides, and silicones.

10. Method according to claim 3 wherein said stent cover is fixed to said stent by a welding process, said welding process comprising heating the PTFE stent cover to a temperature that is greater than the sintering temperature of the PTFE.

## Patentansprüche

1. Verfahren zum Bedecken einer Endoprothesevorrichtung mit einem porösen Produkt aus PTFE ohne Knoten- und Fibrillenstruktur (5; 12; 20), das Verfahren umfasst die Schritte von:
• Bereitstellen eines langgestreckten, radial expandierbaren röhrenförmigen Stents mit einer inneren Oberfläche und einer äußeren Oberfläche, die sich entlang der Längsachse des Stents erstrecken,
• Bereitstellen einer Mischung aus PTFE und einem extrahierbaren Polymermaterial;
• Extrudieren dieser Mischung, um ein Extrudat (40) zu erzeugen, umfassend eine PTFE-Matrix (42) mit diskreten Domänen des extrahierbaren Polymermaterials (44);
• Unterwerfen dieses Extrudates einem Lösungsmittel für das Polymermaterial, einer Temperatur, die ausreicht, das Polymermaterial abzubauen, oder einer Kombination daraus, wobei das Extrudat in der Form eines interpenetrierenden Netzwerks aus Polytetrafluorethylen und dem extrahierbaren Polymermaterial vorliegt und das extrahierbare Material Siloxan ist, wodurch mindestens ein Teil des Polymermaterials extrahiert wird und **dadurch** in dem nicht gestreckten Extrudat (30) poröses Polytetrafluorethylen mit Poren (25) ohne eine Knoten- und Fibrillenstruktur erzeugt wird,
• Erzeugen eines Stentüberzugs aus dem porösen PTFE; und
• Aufbringen des Stentüberzugs auf die innere Oberfläche, äußere Oberfläche oder beide von dem Stent, wobei der Stentüberzug sich entlang der Längsachse des Stents erstreckt.

2. Verfahren gemäß Anspruch 1, wobei die Mischung als eine Röhre extrudiert wird.

3. Verfahren gemäß Anspruch 1, wobei die Mischung als ein Film extrudiert wird, welcher nachfolgend zu einer Röhre gewickelt wird.

4. Verfahren gemäß Anspruch 1, wobei das Siloxan durch eine Verbindung ausgewählt aus der Gruppe bestehend aus Toluol, Heptan und Chloroform chemisch extrahiert wird.

5. Verfahren gemäß Anspruch 1, wobei das Siloxan durch Erwärmen des Netzwerks auf eine Temperatur von mindestens etwa 300°C entfernt wird.

6. Verfahren gemäß Anspruch 1, wobei das Siloxan Polydimethylsiloxan ist.

7. Verfahren gemäß Anspruch 1, wobei der Stentüberzug auf die innere Oberfläche und auf die äußere Oberfläche des Stents aufgebracht wird.

8. Verfahren gemäß Anspruch 1, wobei der Stentüberzug unter Verwendung eines Klebstoffs an dem Stent befestigt wird.

9. Verfahren gemäß Anspruch 8, wobei der Klebstoff aus der Gruppe bestehend aus Polyurethanen, Epoxidharzen, Cyanacrylaten, Polyamiden, Polyimiden und Siliconen ausgewählt ist.

10. Verfahren gemäß Anspruch 3, wobei der Stentüberzug durch einen Schweißvorgang an dem Stent befestigt wird und dieser Schweißprozess umfasst, den Stentüberzug aus PTFE auf eine Temperatur zu erwärmen, die höher als die Sintertemperatur von PTFE ist.

## Revendications

1. Procédé pour revêtir un dispositif d'endoprothèse avec un produit poreux de PTFE sans structure de fibrilles et de noeuds (5; 12; 20), le procédé comportant les étapes de:
• fournir un Stent tubulaire longitudinal radialement expansible ayant une surface intérieure et une surface extérieure, qui s'étendent le long de l'axe longitudinal du Stent,
• fournir un mélange de PTFE et un matériau polymère lessivable;
• extruder ce mélange pour former un objet extrudé (40) qui comporte une matrice de PTFE (42) avec des domaines discrètes du matériau polymère lessivable (44);
• soumettre cet objet extrudé à un solvant pour ledit matériau polymère, à une température qui suffit pour dégrader le matériau polymère, ou à une combinaison de ceux-ci, l'objet extrudé étant dans la forme d'un réseau interpénétrant de polytétrafluor éthylène et le matériau polymère lessivable, le matériau lessivable étant de la siloxane, par lequel au moins une part du matériau polymère est lessivée, et ainsi se forme de la polytétrafluor éthylène poreuse avec des pores (25) dans l'objet extrudé non étiré (30) sans une structure de fibrilles et de noeuds,
• former un revêtement de Stent du ledit PTFE poreux; et
• appliquer ledit revêtement de Stent à la surface intérieure, à la surface extérieure ou les deux du Stent, le revêtement de Stent s'étendant le long de axe longitudinal du Stent.

2. Procédé selon la revendication 1, dans lequel le mélange est extrudé comme un tube.

3. Procédé selon la revendication 1, dans lequel le mélange est extrudé comme un film qui est ensuite enroulé en tube.

4. Procédé selon la revendication 1, dans lequel le siloxane est chimiquement lessivé par un composé sélectionné du groupe consistant de toluène, heptane et chloroforme.

5. Procédé selon la revendication 1, dans lequel le siloxane est éliminé par échauffement du réseau à une température d'au moins environ 300°C.

6. Procédé selon la revendication 1, dans lequel le siloxane est du polydimethylsiloxane.

7. Procédé selon la revendication 1, dans lequel le revêtement de Stent est appliqué sur la surface intérieure et sur la surface extérieure du Stent.

8. Procédé selon la revendication 1, dans lequel le revêtement de Stent est fixé au Stent en utilisant une colle.

9. Procédé selon la revendication 8, dans lequel la colle est sélectionnée du groupe consistant de polyuréthanes, résines époxy, cyanoacrylates, polyamides, polyimides et silicones.

10. Procédé selon la revendication 3, dans lequel le revêtement de Stent est fixé au Stent par un processus de soudage, ledit processus de soudage comportant l'échauffement du revêtement de Stent en PTFE à une température qui est plus haute que la température de frittage du PTFE.
